# FASCICULE DE BREVET EUROPEEN

(11) **EP 4 079 356 B1**
(45) Date de publication et mention de la délivrance du brevet: **15.05.2024**
(21) Numéro de dépôt: 22156107.9
(22) Date de dépôt: 10.02.2022
(51) Int. Cl.: A61M 16/00

(54) **APPAREIL À TOUX METTANT EN OEUVRE UNE ANIMATION VIDÉO POUR AIDER LE PATIENT À EFFECTUER SON TRAITEMENT, EN PARTICULIER EN MODE IPPB**
HUSTENGERÄT MIT VIDEOANIMATION ZUR UNTERSTÜTZUNG DES PATIENTEN BEI DER DURCHFÜHRUNG SEINER BEHANDLUNG, INSBESONDERE IM IPPB-MODUS
COUGH ASSIST MACHINE USING A VIDEO ANIMATION FOR ASSISTING THE PATIENT WITH PERFORMING THEIR TREATMENT, IN PARTICULAR IN IPPB MODE

(30) Priorité: 23.04.2021 FR 2104249
(43) Date de publication de la demande: 26.10.2022
(73) Titulaire: Air Liquide Medical Systems, 92182 Antony Cedex (FR); EOVE, 64000 Pau (FR)
(72) Inventeur: LEROUX, Jean, 92182 Antony Cedex (FR); GALBRUN, Marie-Amédée, 64000 Pau (FR)
(74) Mandataire: Air Liquide

(56) Documents cités:
- EP-A1- 3 622 991
- WO-A2-2010/058308
- US-A1- 2007 199 566

## Description

La présente invention concerne un appareil d'assistance à la toux ou appareil à toux permettant d'opérer au moins des insufflations de gaz à un ou des patients souffrant de troubles respiratoires nécessitant une aide pour évacuer de leurs sécrétions pulmonaires et/ou pour améliorer leur élasticité pulmonaire et thoracique, que ces patients soient des patients adultes ou pédiatriques, lequel peut être utilisé facilement par une personne n'ayant pas ou peu de connaissances médicales, typiquement une auxiliaire de vie, une personne de l'entourage familial du patient, encore appelés « les aidants ».

Les dispositifs médicaux appelés « assistants de toux », « appareils d'assistance à la toux », « appareil à toux » ou analogue, sont des appareils complémentaires aux respirateurs médicaux, qui permettent de générer une pression gazeuse contrôlée afin d'aider un patient à tousser, expectorer et se désencombrer.

Le principe d'un assistant de toux est d'aider à la mobilisation et à l'expectoration des sécrétions bronchiques en gonflant les poumons avec une pression positive puis en appliquant une pression négative pour faciliter le déplacement des mucosités. De tels appareils assistants de toux sont notamment décrits par EP-A-3622991, EP-A-3622992 et EP-A-862922. Un assistant de toux peut aussi être utilisé pour tenter d'améliorer l'élasticité pulmonaire et thoracique, chez certains patients.

Les dispositifs d'assistance à la toux comprennent généralement une turbine motorisée, aussi appelée compresseur ou micro-soufflante, tournant à vitesse fixe ou variable, couplée à une ou plusieurs électrovannes permettant d'orienter le flux d'air depuis la turbine vers le patient, lors des phases d'insufflation de gaz, puis depuis le patient vers la turbine, lors des phases d'exsufflation. Le patient est donc soumis à une alternance de phases d'insufflation (IN) et d'exsufflation (EX) de gaz.

Lors des phases d'insufflation, la sortie de turbine est connectée fluidiquement au patient, alors que, lors des phases d'exsufflation, l'entrée d'air de la turbine est connectée fluidiquement au patient afin d'assurer une aspiration, voire une dépression dans certains cas. Des capteurs de pression et de débit coopérant avec des moyens de pilotage permettent de gérer les différentes phases en fonction des réglages choisis, en particulier des modes ventilatoires. Un oscillateur de gaz peut également être prévu pour augmenter la capacité du dispositif à mobiliser les sécrétions pulmonaires ou bronchiques des patients traités.

La manipulation de ces appareils est habituellement confiée à un professionnel de santé, principalement les kinésithérapeutes. Ceci implique donc un déplacement systématique du professionnel de santé au domicile de chaque patient, jusqu'à plusieurs fois par jour, lorsque plusieurs séances quotidiennes sont nécessaires. De plus, le traitement du patient ne se fait pas forcément au meilleur moment pour lui car il dépend essentiellement du passage du professionnel de santé. Il serait donc souhaitable de pouvoir déclencher une séance d'utilisation de l'appareil à toux au meilleur moment pour le patient de manière à la rendre plus efficace et par ailleurs à réduire considérablement le stress du patient et à éviter des déplacements pouvant être fréquents du personnel de santé.

De plus, il peut être compliqué pour certains patients, notamment les patients pédiatriques (i.e. enfants...), d'utiliser correctement l'appareil à toux lorsqu'un kinésithérapeute ou analogue n'est pas présent pour les aider, car ils doivent réussir à synchroniser leur respiration avec les phases d'insufflation et d'exsufflation de gaz par la turbine de l'appareil à toux, pour que le traitement soit efficace.

Par ailleurs, US-A-2007/0199566 enseigne un ventilateur médical permettant de réaliser des insufflations et exsufflations de gaz à un patient. Un afficheur graphique permet d'afficher une représentation des poumons du patient dont la taille augmente ou diminue en fonction de mesures d'un paramètre thoracique du patient, ce qui permet de suivre les inspirations et expirations du patient au moment où elles se produisent, c'est-à-dire que la représentation des poumons est une image de la respiration du patient lorsqu'elle se produit. Autrement dit, l'affichage du mouvement des poumons reflète uniquement la manière de respirer du patient mais ne constitue pas une aide permettant au patient de synchroniser sa respiration avec les phases d'insufflation et d'exsufflation de gaz par la turbine de l'appareil à toux. D'ailleurs, cet affichage nécessite d'opérer des mesures sur le patient.

De là, le problème est de proposer un appareil d'assistance à la toux pouvant fonctionner simplement et être mis en oeuvre par une personne moins formée qu'un kinésithérapeute, par exemple une auxiliaire de vie, une personne de l'entourage familial du patient... de sorte à pouvoir déclencher chez un patient, c'est-à-dire débuter, une séance d'utilisation de l'appareil à toux, au moment le plus opportun pour le patient considéré, et sans avoir à faire venir spécialement un professionnel de santé qualifié de type kinésithérapeute ou analogue, tout en obtenant un traitement aussi efficace qu'en présence d'un tel kinésithérapeute et/ou en rendant le patient adhérent ou plus adhérent à son traitement, en particulier dans le cas où il convient d'améliorer l'élasticité pulmonaire et/ou thoracique du patient.

L'invention concerne alors un appareil à toux pour opérer au moins une insufflation de gaz à un patient, comprenant une turbine motorisée en communication fluidique avec un circuit de gaz comprenant une ligne d'insufflation et une ligne d'exsufflation, un écran d'affichage et des moyens de pilotage configurés pour commander la turbine et/ou un affichage sur l'écran d'affichage, et dans lequel les moyens de pilotage sont configurés pour afficher sur l'écran d'affichage, une animation vidéo mettant en oeuvre une représentation graphique symbolisant un patient pendant au moins une phase d'insufflation de gaz.

Il comprend en outre un capteur de débit agencé sur le trajet du gaz en aval de la sortie de la turbine et configuré pour opérer des mesures permettant de déterminer le débit de gaz fourni par la turbine, lesdites mesures opérées par le capteur de débit étant traitées par les moyens de pilotage afin d'en déduire un volume de gaz délivré par la turbine.

La forme, l'aspect et/ou les dimensions de la représentation graphique affichée sur l'écran d'affichage varient en corrélation avec le volume de gaz fourni par la turbine au patient, pendant ladite au moins une phase d'insufflation. Dès lors, les moyens de pilotage de l'appareil sont configurés pour :
a) commander un démarrage de la turbine et simultanément de l'animation vidéo en réponse à une activation par l'utilisateur de moyens de démarrage d'insufflation ou à une détection d'une inspiration de l'utilisateur, et
b) stopper la turbine et simultanément l'animation vidéo lorsqu'une pression maximale d'insufflation est atteinte ou à la fin d'une durée maximale d'insufflation.

Autrement dit, la représentation graphique qui s'affiche sur l'écran d'affichage représente le volume de gaz progressivement délivré par la turbine, donc que le patient a réussi à inhaler pendant la phase d'inspiration, c'est-à-dire à faire entrer dans ses poumons pendant son traitement. Cette représentation graphique n'est donc pas obtenue en mesurant un paramètre thoracique du patient mais est basée sur le volume que fournit la turbine. Elle sert en fait à encourager le patient à prendre encore plus de volume gazeux dans ses poumons par rapport à ce qu'il a déjà réussi à y faire entrer.

Ceci permet d'aider et motiver le patient à maintenir son inspiration de gaz pendant toute la durée de la (chaque) phase d'insufflation de gaz opérée par la turbine de l'appareil à toux de manière à ce qu'il inhale, si possible, tout le volume de gaz fourni, en particulier en mode IPPB comme expliqué ci-après.

Ainsi, le patient n'a qu'à se baser et/ou à calquer sa respiration sur la ou les variations de forme, d'aspect et/ou de dimensions de la représentation graphique affichée sur l'écran d'affichage de manière à continuer à inspirer tant que la représentation graphique varie. De cette manière, on peut améliorer l'élasticité pulmonaire et/ou thoracique du patient grâce à la pression gazeuse appliquée à ses poumons, voire aussi provoquer un recrutement alvéolaire et/ou une mobilisation des sécrétions et l'expectoration.

Selon le cas, l'appareil à toux de l'invention peut comprendre l'une ou plusieurs des caractéristiques techniques suivantes :
- l'appareil à toux est configuré pour permettre d'opérer plusieurs insufflations de gaz successives.
- chaque phase d'insufflation est suivie par une phase d'exsufflation de gaz.
- l'animation vidéo met en oeuvre une représentation graphique symbolisant le patient pendant au moins une phase d'insufflation de gaz et éventuellement au moins une phase d'exsufflation de gaz, ladite phase d'exsufflation de gaz étant consécutive à ladite phase d'insufflation de gaz.
- l'animation vidéo comprend une pluralité d'images successives.
- les moyens de pilotage sont configurés pour commander, en réponse à une activation par l'utilisateur d'une touche de démarrage d'insufflation ou d'inhalation, un début de traitement du patient comprenant un démarrage de la turbine et simultanément de l'animation vidéo.
- alternativement, les moyens de pilotage sont configurés pour commander, en réponse à une détection d'une inspiration par l'utilisateur (i.e. début de phase inspiratoire avec inhalation de gaz), un début de traitement du patient comprenant un démarrage de la turbine et simultanément de l'animation vidéo.
- il comprend des moyens de détection d'inspiration comprennent un capteur de pression, de préférence le capteur de pression est agencé dans l'appareil, par exemple au niveau de la sortie de gaz allant vers le patient.
- les moyens de détection d'inspiration coopèrent avec les moyens de pilotage pour leur fournir un signal représentatif d'un début de phase inspiratoire chez le patient.
- le démarrage de la turbine comprend un début de fourniture de volume de gaz par ladite turbine, typiquement d'air.
- les variations de dimensions de la représentation graphique se font de manière synchronisées avec la délivrance progressive du volume de gaz par la turbine.
- les moyens de démarrage/arrêt de traitement comprennent une touche de démarrage/arrêt unique (i.e. une seule et même touche) commandant le démarrage et l'arrêt d'un traitement, de préférence une touche tactile.
- la (ou les) touche tactile est affichée sur l'écran d'affichage, c'est-à-dire que l'écran d'affichage est configuré pour afficher la (ou les) touche tactile.
- la (ou les) touche tactile coopère avec les moyens de pilotage.
- les variations de dimensions de la représentation graphique se font de manière progressive entre le début et la fin des phases d'insufflation et éventuellement d'exsufflation de gaz, c'est-à-dire des phases inspiratoires et/ou éventuellement expiratoires du patient.
- les variations de dimensions de la représentation graphique se font de manière synchronisées avec une variation de couleur de la représentation graphique, en particulier une succession de couleurs, par exemple violet et vert, ou toute autre couleur(s).
- la représentation graphique représente ou comprend un animal ou un personnage, par exemple un caméléon ou tout autre animal, c'est-à-dire un animal ou un personnage symbolisant le patient qui suit le traitement par insufflation et exsufflation de gaz.
- la représentation graphique symbolise au moins une phase d'inspiration du patient, notamment une phase d'inspiration et une phase d'expiration suivant ladite phase d'inspiration, et éventuellement une phase de pause (entre les phases d'inspiration et d'expiration, pendant laquelle le patient ni n'inspire, ni n'expire de gaz.
- les moyens de pilotage sont configurés pour commander la turbine de manière à insuffler progressivement un volume de gaz au patient, typiquement de l'air.
- l'écran d'affichage est configuré pour afficher la vidéo d'animation en couleurs ou en noir et blanc, de préférence en couleurs.
- l'appareil comprend en outre des moyens de mémorisation configurés pour mémoriser la pluralité d'images formant la vidéo d'animation, par exemple une mémoire informatique de type EEPROM ou analogue.
- l'affichage de la vidéo d'animation est opéré en temps réel pendant les phases d'insufflation et d'exsufflation de gaz.
- les moyens de sélection de mode de ventilation comprennent une ou des touches tactiles.
- l'appareil comprend en outre des moyens de sélection d'un mode de ventilation choisi parmi les modes INEX et IPPP, où :
   o le mode INEX correspond à une alternance de phases insufflatoire et exsufflatoire de gaz avec insufflation de gaz à pression positive suivi d'une exsufflation de gaz par mise en dépression, et
   o le mode IPPB correspond à une alternance de phases insufflatoire et exsufflatoire de gaz avec insufflation de gaz jusqu'à atteindre une pression de consigne maximale ou une durée d'insufflation maximale, suivi d'une exsufflation de gaz sans mise en dépression.
- en mode IPPB, les moyens de pilotage de l'appareil sont configurés pour stopper la turbine, donc l'insufflation de gaz, et simultanément l'animation vidéo lorsqu'une pression maximale d'insufflation est atteinte ou à la fin d'une durée maximale d'insufflation.
- les moyens de pilotage sont configurés pour afficher sur l'écran d'affichage et démarrer l'animation vidéo indépendamment de toute mesure de paramètre thoracique du patient, c'est-à-dire en l'absence et sans recours à un tel paramètre thoracique.
- le capteur de débit est agencé sur la ligne d'insufflation.
- les moyens de pilotage sont configurés pour afficher sur l'écran d'affichage l'animation vidéo pour permettre au patient de calquer son ou ses inspirations sur l'animation vidéo.
- l'animation vidéo reflète la quantité de gaz, i.e. le volume de gaz, délivré par la turbine et que le patient a donc inspiré pendant une durée donnée, typiquement pendant la durée d'une inspiration, et ce, en l'absence de toute mesure d'un quelconque paramètre thoracique.
- l'animation vidéo varie en corrélation/synchronisme avec le volume de gaz délivré par la turbine au patient, pendant une phase d'insufflation de gaz, étant donné que turbine et animation vidéo démarrent en même temps, donc indépendamment de toute mesure de paramètre thoracique ou analogue.
- la forme, l'aspect et/ou les dimensions de ladite représentation graphique affichée sur l'écran d'affichage varient uniquement en corrélation avec le volume de gaz fourni par la turbine au patient, c'est-à-dire indépendamment et/ou en l'absence de toute mesure de paramètre thoracique.
- les dimensions de la représentation graphique affichée sur l'écran d'affichage varient proportionnellement au volume de gaz fourni par la turbine au patient.
- les dimensions (e.g. taille) de la représentation graphique affichée sur l'écran d'affichage augmentent proportionnellement à une augmentation progressive du volume de gaz fourni par la turbine au patient et uniquement en fonction de cette augmentation progressive du volume de gaz fourni par la turbine, c'est-à-dire sans avoir recourt à un quelconque paramètre de mesure thoracique opéré sur le patient.
- la forme et/ou l'aspect de la représentation graphique affichée sur l'écran d'affichage varient proportionnellement à une augmentation progressive du volume de gaz fourni par la turbine au patient et uniquement en fonction de cette augmentation progressive du volume de gaz fourni par la turbine, c'est-à-dire sans avoir recourt à un quelconque paramètre de mesure thoracique opéré sur le patient.
- l'écran d'affichage est configuré pour afficher la ou les touches tactiles permettant de sélectionner le mode de ventilation choisi parmi les modes INEX et IPPB.
- les moyens de sélection de mode de ventilation sont configurés pour sélectionner un ensemble de paramètres ou préréglages ventilatoires, aussi appelé programme de ventilation, adapté à la mise en oeuvre d'une ventilation en mode de ventilation INEX ou IPPB.
- le ou les préréglages ventilatoires de chaque mode de ventilation sont fixés ou choisis par un personnel soignant.
- le ou les préréglages de chaque mode de ventilation sont mémorisés.
- le mode INEX correspond à une alternance de phases insufflatoire et exsufflatoire de gaz, éventuellement espacées par une phase de pause, avec insufflation de gaz à pression positive haute assez élevée, par exemple d'au moins 5 mbar, de préférence d'au moins 15 à 20 mbar, par exemple de l'ordre de 30 bar, pendant une durée d'insufflation courte, par exemple de 1 à 3 sec, suivi d'une exsufflation de gaz par mise en dépression (i.e. pression négative), par exemple de l'ordre de -30 mbar, des poumons et de la cage thoracique du patient pour provoquer la mobilisation et l'expulsion/extraction des sécrétions pulmonaires et/ou bronchiques La pression positive haute, la pression négative et les durées d'insufflation et/ou d'exsufflation, et d'autres paramètres sont réglables.
- le mode IPPB correspond à une alternance de phases insufflatoire et exsufflatoire de gaz, éventuellement espacées par une phase de pause, avec insufflation de gaz à débit faible, par exemple compris entre 5 et 100 L/min, par exemple entre 10 et 15 L/min, jusqu'à atteindre une pression de consigne maximale ou une durée d'insufflation maximale, typiquement une pression de consigne maximale entre 10 et 50 mbar, par exemple de l'ordre de 30 à 40 mbar, pendant une durée d'insufflation variable (pouvant atteindre de l'ordre de 10 secondes) et avec fourniture d'un volume de gaz variable, en fonction de l'élasticité des poumons et de la cage thoracique du patient, suivi d'une exsufflation de gaz sans mise en dépression (i.e. pression négative) des poumons et de la cage thoracique du patient, par exemple pendant une durée de 1 à 3 secondes, pour provoquer un recrutement alvéolaire et/ou une mobilisation des sécrétions et l'expectoration et/ou par ailleurs améliorer l'élasticité pulmonaire et/ou thoracique du patient grâce à la pression gazeuse appliquée à ses poumons. Le niveau de pression maximale (i.e. positive), le débit de gaz administré et/ou la durée d'insufflation maximale sont réglables.
- le déroulé de l'affichage de la vidéo d'animation est synchronisé avec le volume de gaz insufflé, lors des phases d'insufflation de gaz, en particulier en mode IPPB.
- le déroulé de l'affichage de la vidéo d'animation est opéré indépendamment de toute mesure de paramètre thoracique.
- les moyens de pilotage sont configurés pour stopper, en réponse à une activation par l'utilisateur de moyens de démarrage/arrêt de traitement, la turbine et simultanément de l'animation vidéo, c'est-à-dire un arrêt de traitement du patient, de préférence une (ou des) touche tactile affichée sur l'écran.
- la vidéo d'animation comprend plusieurs images différentes successives comprenant la représentation graphique dont la forme, l'aspect (y compris la couleur) et/ou les dimensions affichées sur l'écran d'affichage varient en corrélation avec les volumes de gaz lors des phases d'insufflation, en particulier en mode IPPB.
- l'écran d'affichage comprend en outre des moyens de sélection d'un sous-mode de fonctionnement, de préférence une (ou des) touche tactile affichée sur l'écran, permettant de (i.e. configurés pour) sélectionner un sous-mode de fonctionnement choisi parmi les modes automatique (AUTO) et manuel.
- l'écran d'affichage est configuré pour afficher en outre, en mode IPPB, un volume de gaz courant, un volume de gaz cible, une durée de séance et/ou un nombre de cycle effectués.
- les moyens de mémorisation sont en outre configurés pour enregistrer, i.e. mémoriser, les préréglages (i.e. ensemble de paramètres) correspondant aux modes INEX et IPPB.
- les préréglages comprennent des paramètres de ventilation adaptés aux modes INEX et IPPB.
- les préréglages comprennent en particulier une ou des valeurs de débit inspiratoire et/ou de pression d'inspiration maximale, un temps maximal d'inspiration, un seuil de déclenchement ou 'trigger', ou autre.
- les préréglages sont fixés par un personnel soignant, par exemple un médecin ou un kinésithérapeute, et mémorisés au sein des moyens de mémorisation préalablement au déroulé d'une séance de thérapie.
- les moyens de pilotage sont en outre configurés pour retrouver au sein des moyens de mémorisation, au moins un préréglage donné adapté à la mise en oeuvre d'un mode de ventilation INEX ou IPPB en réponse à une sélection par un utilisateur, via les moyens de sélection de mode de ventilation, d'un desdits modes de ventilation INEX ou IPPB.
- il comprend au moins une touche pour régler ou sélectionner un ou des préréglages adaptés aux modes de ventilation INEX et IPPB, de préférence une touche tactile affichée sur l'écran.
- les moyens de pilotage sont configurés pour commander la turbine de manière à délivrer du gaz pendant les phases d'insufflation et éventuellement d'exsufflation de gaz au patient.
- il comprend une carcasse rigide.
- la turbine motorisée, la ligne d'insufflation, la ligne d'exsufflation et les moyens de pilotage sont agencés dans la carcasse.
- l'écran d'affichage est porté par la carcasse.
- l'écran d'affichage est fixé, i.e. solidarisé, de manière détachable ou non détachable à la carcasse.
- il comprend une interface homme-machine (IHM), aussi appelée interface graphique utilisateur (IGU), configurée pour permettre à un utilisateur d'opérer une ou des sélections ou des choix, de rentrer ou d'ajuster/modifier une ou des valeurs de consigne, notamment des valeurs de pression haute et basse, de temps des différentes phases...
- l'écran d'affichage fait partie de l'IHM.
- l'écran d'affichage est un écran numérique tactile, typiquement à affichage en couleurs ou en noir et blanc.
- l'écran d'affichage comprend en outre une ou des touches tactiles permettant d'opérer des sélections, des validations, des réglages, des démarrages ou des arrêts de fonctionnement... Les touches tactiles sont à actionnement digital, c'est-à-dire qu'elles sont activées lorsque l'utilisateur appuie dessus avec son doigt, typiquement son index.
- l'écran d'affichage est configuré pour opérer un affichage d'informations sous forme de caractères alphanumériques, de représentations graphiques (e.g. graphes, courbes, dessins, icones, etc....), de photos, d'animations vidéo ou autres.
- la ligne d'insufflation est raccordée fluidiquement à la sortie de gaz de la turbine.
- la ligne d'exsufflation est accordée fluidiquement à l'entrée de gaz de la turbine.
- une ligne commune de fourniture de gaz à un patient raccordée fluidiquement auxdites ligne d'insufflation et ligne d'exsufflation.
- il comprend des valves pneumatiques agencées sur la ligne d'exsufflation et sur la ligne d'insufflation et des moyens de commande pneumatiques commandant pneumatiquement lesdites valves pneumatiques.
- les valves pneumatiques agencées sur la ligne d'exsufflation sont configurées pour contrôler une mise en communication fluidique de la ligne d'exsufflation avec l'atmosphère et/ou une mise en communication fluidique de la ligne commune de fourniture de gaz avec la ligne d'exsufflation.
- les valves pneumatiques agencées sur la ligne d'insufflation sont configurées pour contrôler une mise en communication fluidique de la ligne d'insufflation avec l'atmosphère et/ou une mise en communication fluidique de la ligne d'insufflation avec la ligne commune de fourniture de gaz.
- la turbine est configurée pour délivrer de l'air.
- une (chaque) phase d'insufflation a une durée comprise entre 0.5 et 20 secondes, typiquement jusqu'à 3 à 8 sec environ, par exemple de l'ordre de 6 sec.
- une (chaque) phase d'exsufflation a une durée comprise entre 0.5 et 10 secondes, typiquement jusqu'à 3 à 5 secondes environ.
- les moyens de pilotage sont configurés pour commander la turbine de manière à opérer, i.e. déclencher, cycliquement une (les) phase d'insufflation.
- de préférence, la (les) phase d'exsufflation est passive et suit une phase d'insufflation.
- optionnellement, la (chaque) phase d'insufflation et la (chaque) phase d'exsufflation qui la suit sont elles-mêmes suivies par une phase de pause, i.e. située entre une phase d'exsufflation et la phase d'insufflation suivante. De préférence, les durées des phases d'exsufflation et/ou de pause ne sont pas paramétrables, i.e. non-réglables, en mode IPPB
- pendant la (chaque) phase de pause, la turbine est commandée par les moyens de pilotage pour délivrer une pression de pause supérieure ou égale à 0 mbar dans la ligne d'insufflation, de préférence une pression de pause entre 0 et 30 mbar, par exemple de l'ordre de 10 à 20 mbar.
- la (chaque) phase de pause a une durée qui dépend du patient et de son désir d'opérer une pause ou non, avant une nouvelle inspiration de gaz.
- les moyens de pilotage comprennent un (micro)contrôleur numérique relié électriquement à la turbine.
- les moyens de pilotage comprennent un microprocesseur, de préférence porté par une carte électronique.
- la turbine comprend un moteur électrique, c'est-à-dire fonctionnant grâce à un courant électrique.
- la turbine comprend un moteur électrique entraînant une roue à ailettes agencée dans le compartiment interne d'une volute.
- pendant une phase d'insufflation et/ou une phase de pause éventuelle, la turbine délivre de l'air sous pression (i.e. > à la pression atmosphérique) dans la ligne d'insufflation raccordée fluidiquement à la sortie de gaz de la turbine, c'est-à-dire que la turbine génère une pression positive (P+) dans ladite ligne d'insufflation. L'air sous pression y chemine en direction de la ligne commune de fourniture de gaz et donc du patient.
- pendant une (chaque) phase d'insufflation en mode IPPB, la turbine génère une pression positive (P+) comprise entre 5 et 70 mbar (pression relative par rapport à la pression atmosphérique), de préférence une pression d'insufflation réglable inférieure ou égale à 50 mbar.
- pendant une (chaque) phase de pause éventuelle en mode IPPB, la turbine génère une pression positive (P+) comprise entre 0 et 30 mbar (pression relative par rapport à la pression atmosphérique) de préférence une pression d'insufflation réglable entre 1 et 20 mbar.
- pendant une (chaque) phase d'exsufflation en mode IPPB, le patient expire seul sans mise en dépression de ses poumons, c'est-à-dire naturellement.
- la turbine est commandée pour atteindre une vitesse de rotation maximale de 75 000 tours/minute, typiquement entre 10 000 et 50 000 tr/min.
- il comprend des moyens d'alimentation en courant électrique alimentant en courant électrique au moins la turbine motorisée, l'IHM et les moyens de pilotage et, éventuellement, la pompe ou le compresseur.
- les moyens d'alimentation en courant électrique comprennent au moins une batterie, de préférence rechargeable, et/ou une prise électrique et un cordon électrique de raccordement au secteur (e.g. 110/230 V), et éventuellement un transformateur de courant.
- le circuit de gaz, en particulier la ligne commune de fourniture de gaz, est relié fluidiquement au patient via une interface respiratoire, tel un masque respiratoire, par exemple un masque bucco-nasal, ou un embout buccal.
- la ligne commune de fourniture de gaz est reliée fluidiquement à l'interface respiratoire par l'intermédiaire d'un tuyau flexible ou analogue.
- l'écran d'affichage est configuré pour afficher la représentation graphique dont la forme, l'aspect et/ou les dimensions varient en corrélation avec le volume de gaz fourni par la turbine au patient, pendant ladite au moins une phase d'insufflation de gaz.

L'appareil à toux de l'invention est adapté au traitement des patients adultes, y compris les personnes âgées, mais aussi pédiatriques, c'est-à-dire les enfants ou les adolescents.

L'invention va maintenant être mieux comprise grâce à la description détaillée suivante, faite à titre illustratif mais non limitatif, en référence aux figures annexées parmi lesquelles :
- Fig. 1 est un schéma d'une architecture interne d'un appareil d'insufflation et d'exsufflation de gaz,
- Fig. 2 schématise un mode de réalisation de l'écran d'affichage d'un appareil à toux selon l'invention avant le démarrage d'un traitement, en mode IPPB, et
- Fig. 3 schématise l'écran d'affichage de Fig. 2 lors d'une insufflation de gaz en mode IPPB.

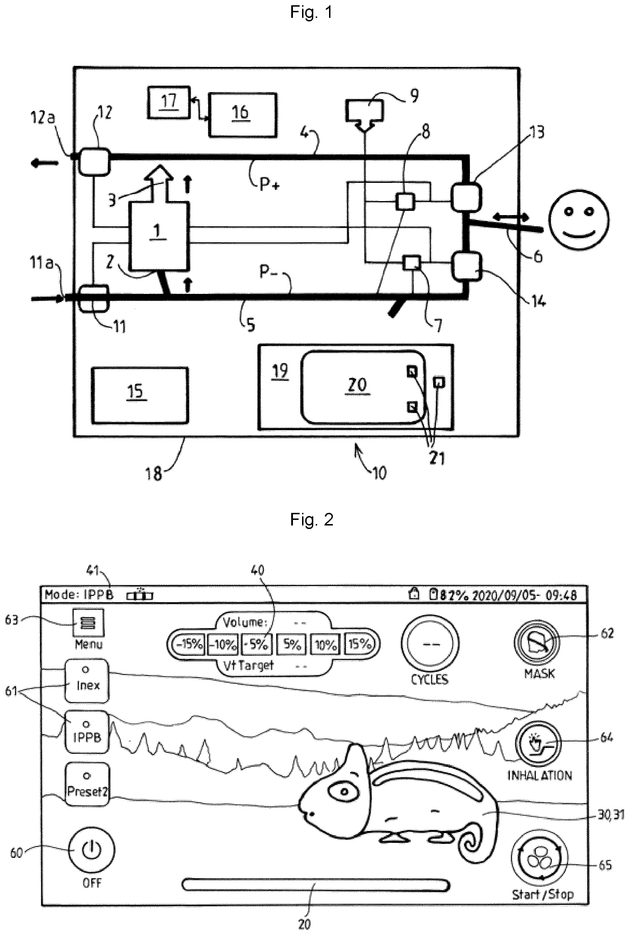

Fig. 1 schématise une architecture interne d'un appareil d'assistance à la toux 10 ou appareil à toux, tel celui de l'invention, permettant de réaliser des insufflations de gaz à un patient souffrant de troubles respiratoires nécessitant une aide à l'évacuation des sécrétions pulmonaires et/ou pour améliorer son élasticité pulmonaire et/ou thoracique, en particulier selon un mode IPPB. Cette architecture est classique et décrite en détail par EP-A-3622991 auquel on peut se reporter pour plus de détail.

Schématiquement, l'appareil à toux 10 comprend une turbine 1 motorisée, aussi appelé compresseur ou (micro)soufflante, comprenant une entrée de gaz 2 par laquelle l'air est aspiré et pénètre dans la turbine 1 et une sortie de gaz 3 par laquelle l'air, de préférence à pression positive, est expulsé et ressort de la turbine 1. La turbine 1 comprend classiquement un moteur électrique entraînant une roue à ailettes agencée dans le compartiment interne de la volute et servant à délivrer de l'air sous pression (> 1 bar).

Il est également prévu des moyens de pilotage 16 commandant notamment la turbine 1, via une liaison électrique, pour délivrer du gaz pendant au moins des phases d'insufflation et d'exsufflation.

Chaque séquence d'insufflation/d'exsufflation peut être suivie par une phase de pause si le patient le souhaite, située entre une phase d'exsufflation et la phase d'insufflation suivante. Pendant la phase de pause, la turbine 1 est commandée par les moyens de pilotage 16 pour délivrer une pression gazeuse comprise par exemple entre 0 et 20 mbar dans la ligne d'insufflation 4 et la ligne commune 6.

De préférence, les moyens de pilotage 16 comprennent un ou plusieurs microprocesseurs, typiquement un contrôleur numérique relié électriquement à la turbine 1. Le contrôleur numérique peut comprendre par exemple une carte électronique à microcontrôleur mettant en oeuvre un ou plusieurs algorithmes servant à piloter la turbine 1 et des moyens de mémorisation, telle une mémoire flash ou autre.

Pendant le fonctionnement de l'appareil 1, la turbine 1 alimente en air, une ligne d'insufflation 4 qui est raccordée fluidiquement à la sortie de gaz 3 de la turbine 1 et permet d'acheminer l'air expulsé par la turbine 1. Par ailleurs, une ligne d'exsufflation 5 est quant à elle raccordée fluidiquement à l'entrée de gaz 2 de la turbine 1 et permet notamment d'y acheminer l'air aspiré par la turbine 1. Les lignes d'insufflation 4 et d'exsufflation 5 sont par exemple des conduits de gaz, des passages de gaz ou analogues, agencés dans l'appareil à toux 10.

Les lignes d'insufflation 4 et d'exsufflation 5 sont par ailleurs raccordées fluidiquement à une ligne commune 6 de fourniture de gaz, tel un conduit de gaz ou analogue, qui est reliée fluidiquement à un patient, par exemple via un tuyau flexible relié fluidiquement à une interface respiratoire, tel qu'un masque respiratoire ou analogue, de sorte d'alimenter les voies respiratoires du patient, notamment ses poumons, en air sous pression pendant les phases d'insufflation et, à l'inverse, d'en extraire le gaz pendant les phases d'exsufflation et à l'aider ainsi à expulser ses sécrétions pulmonaires ou bronchiques.

Les lignes d'insufflation 4 et d'exsufflation 5 comprennent plusieurs valves pneumatiques 11, 12, 13, 14 permettant de contrôler les flux gazeux pendant les phases d'insufflation et d'exsufflation successives, et optionnellement pendant les phases de pause et/ou transitoires.

Schématiquement, une première 11 et une quatrième 14 valves pneumatiques sont agencées sur la ligne d'exsufflation 5. La première valve pneumatique 11 contrôle la mise en communication fluidique de la ligne d'exsufflation 5 avec l'atmosphère ambiante, via un orifice d'entrée 11a, pour permettre à de l'air atmosphérique de d'y pénétrer et d'alimenter la turbine 1, pendant les phases d'insufflation, alors que la quatrième valve pneumatique 14 contrôle la mise en communication fluidique de la ligne commune 6 de fourniture de gaz avec la ligne d'exsufflation 5, pendant les phases d'exsufflation.

Par ailleurs, une deuxième 12 et une troisième 13 valves pneumatiques sont agencées sur la ligne d'insufflation 4. La deuxième valve pneumatique 12 contrôle la mise en communication fluidique de la ligne d'insufflation 4 avec l'atmosphère, via un orifice d'évacuation de gaz 12a pour évacuer le gaz sous pression, pendant les phases d'exsufflation, comme expliqué ci-après, alors que la troisième valve pneumatique 13 contrôle la mise en communication fluidique de la ligne d'insufflation 4 avec la ligne commune 6 de fourniture de gaz, pour alimenter le patient en air, pendant les phases d'insufflation.

Ces valves pneumatiques 11-14 sont elles-mêmes pilotées pneumatiquement par des moyens de commande pneumatiques 7, 8 comprenant des première et seconde électrovannes pneumatiques 7, 8, à savoir des électrovannes miniatures à faible consommation de pic (i.e. < 10 Watt) qui sont pilotées électriquement, via une ou plusieurs liaisons électriques, par les moyens de pilotage 16 de l'appareil à toux 10, en fonction de la phase d'insufflation ou d'exsufflation à opérer, et/ou des éventuelles phases de pause.

Les première et seconde électrovannes pneumatiques 7, 8 sont par ailleurs reliées pneumatiquement à au moins une ligne de pilotage pneumatique, c'est-à-dire au moins une ligne d'amenée de pression positive, par exemple une conduite de gaz qui se ramifie ou plusieurs conduites, reliant ces première et seconde électrovannes pneumatiques 7, 8 à une source de pression positive 9, i.e. une source de gaz sous pression, tel qu'une micro-pompe ou micro-compresseur additionnel.

En outre, les première et seconde électrovannes 7, 8 sont aussi connectées fluidiquement à une source de pression dite « négative », appelée simplement source de pression négative, via au moins une ligne d'amenée de pression négative, par exemple à la ligne d'exsufflation 5 où règne une pression négative (P-) pendant les phases d'exsufflation.

Le fonctionnement et la structure de ces valves pneumatiques et électrovannes sont détaillés dans EP-A-3622991, auquel on peut se reporter pour plus de détail.

Un tel appareil peut être utilisé pour mettre en oeuvre des modes INEX, IPPB ou autres ; toutefois, seul le mode IPPB est décrit en détail ci-après.

Ainsi, en mode IPPB, l'insufflation de gaz par la turbine 1 s'effectue à débit faible, par exemple de l'ordre de 5 à 100 mL/Min, pendant une durée d'insufflation réglable pouvant atteindre environ 20 sec, par exemple de l'ordre de 6 sec, pendant laquelle du gaz est insufflé jusqu'à atteindre une pression de consigne donnée ou la fin de la durée d'insufflation préfixée, pour faire varier, i.e. augmenter, progressivement et de manière douce l'élasticité de la cage thoracique et des poumons du patient, suivie d'une exsufflation ou sortir de gaz des poumons du patient jusqu'à ce que la cage thoracique et les poumons du patient reviennent à leur état de repos et ce, de manière passive, c'est-à-dire sans avoir recours à une mise en dépression.

Autrement dit, pendant une phase d'insufflation, la turbine 1 génère une pression positive (P+) du côté de la sortie 3 de gaz de la turbine 1, c'est-à-dire une surpression, dans la ligne d'insufflation 4 et dans la ligne commune 6 de fourniture de gaz qui est fluidiquement raccordée à la ligne d'insufflation 5, pendant une durée d'insufflation assez longue, i.e. jusqu'à environ 20 secondes, du fait du débit de gaz faible appliqué jusqu'à atteindre pression de consigne maximale ou une durée maximale.

Ensuite, pendant la phase d'exsufflation qui suit la phase d'insufflation, le gaz expiré par le patient chemine successivement dans la ligne commune 6 et dans la ligne d'exsufflation 5, ce qui permet d'évacuer naturellement le gaz contenu dans le tractus respiratoire du patient, en particulier dans ses poumons, en engendrant un retour naturel de la cage thoracique et des poumons du patient à leur état de repos, c'est-à-dire que les poumons se vident tout seul sans assistance de la turbine, en mode IPPB.

Le gaz aspiré est ensuite évacué vers l'atmosphère ambiante, au travers de la turbine 1, via l'orifice de mise à l'atmosphère 12a de la deuxième valve pneumatique 12.

Le débit de gaz, la durée maximale d'inspiration et la pression maximale d'inspiration, c'est-à-dire la pression de consigne maximale, et d'autres paramètres ventilatoires utiles, sont réglables en mode IPPB. Ils sont réglés ou fixés par un personnel soignant, tel un médecin ou un kinésithérapeute, puis mémorisés sous forme de préréglages, c'est-à-dire d'ensembles de paramètres respiratoires correspondant aux modes ventilatoires INEX et IPPB.

Pendant les phases d'insufflation, la turbine 1 est commandée par les moyens de pilotage 16. La vitesse de rotation maximale de la turbine peut atteindre environ 75 000 tours/minute, typiquement entre 10 000 et 50 000 tr/min.

Les éléments de l'appareil à toux 10 montrés sur la Fig. 1 peuvent être agencés dans une carcasse 18 ou coque externe rigide, par exemple une carcasse en polymère ou analogue.

Une interface homme-machine 19 ou IHM, agencée sur la carcasse 18, permet à l'utilisateur de rentrer des valeurs de consigne dans l'appareil à toux 10, d'opérer des sélections ou des choix...

L'IHM 19 comprend un écran tactile 20, de préférence à affichage en couleurs, et des touches 21 de sélection ou autres, notamment numériques tactiles s'affichant sur l'écran tactile 20, i.e. écran digital.

Des moyens d'alimentation 15 en courant électrique (non montrés) alimentent en courant électrique les différents composants de l'appareil à toux 10 nécessitant de l'être, à savoir notamment la turbine 1 motorisée, les moyens de pilotage 16, l'IHM 19, l'écran d'affichage 20... Les moyens d'alimentation 15 en courant électrique comprennent par exemple une (ou plusieurs) batterie, de préférence rechargeable, et/ou une prise électrique et un cordon électrique (non montré) de raccordement au secteur, e.g. 110/230 V, avec ou sans transformateur de courant.

D'une manière générale, un appareil à toux 10 selon la présente invention est destiné à être utilisé pour opérer des insufflations de gaz (i.e. d'air) à des patients incapables de gérer seuls leurs sécrétions et/ou pour améliorer leur élasticité pulmonaire et thoracique, que ces patients soient des patients adultes ou pédiatriques.

L'appareil à toux 10 est préférentiellement suffisamment léger et compact pour être facilement transportable. Il peut être utilisé à domicile ou à l'hôpital, avec des interfaces respiratoires invasives (e.g. sondes trachéales) ou non-invasives (e.g. masques).

Les moyens de pilotage 16 sont aussi configurés pour commander l'affichage d'informations de toute nature, à savoir des caractères alphanumériques, des icônes, des représentations graphiques, des courbes ou autres, sur l'écran d'affichage 20 de l'IHM 19, lequel opère alors un affichage de ces informations de manière à assister, informer ou autre, l'utilisateur.

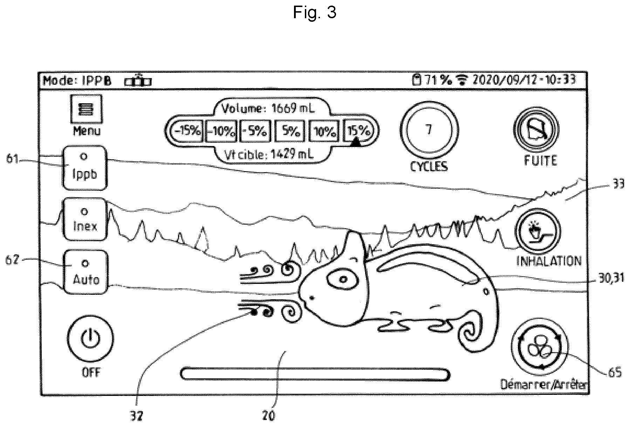

Selon la présente invention, afin de permettre l'utilisation de l'appareil à toux 10 par une personne moins formée qu'un kinésithérapeute, par exemple une auxiliaire de vie, une personne de l'entourage familial du patient... afin de pouvoir déclencher chez le patient, une séance d'utilisation de l'appareil à toux 10, au moment le plus opportun pour le patient considéré, sans avoir à faire venir spécialement un professionnel de santé qualifié de type kinésithérapeute ou analogue, et à attendre son passage, les moyens de pilotage 16 sont configurés pour commander un affichage simultané, sur l'écran d'affichage 20 tactile, une animation vidéo mettant en oeuvre une représentation graphique 30 symbolisant le patient qui doit être traité, et dans laquelle la forme, l'aspect et/ou les dimensions de cette représentation graphique 30 affichée sur l'écran d'affichage 20 varient en corrélation avec les phases d'insufflation par la turbine 2 de l'appareil à toux 10, et préférentiellement d'exsufflation de gaz, comme illustré sur les Fig. 2 et Fig. 3.

Dans le mode de réalisation illustré sur ces Fig. 2 et Fig. 3, concernant le mode IPPB, la représentation graphique 30 symbolisant le patient, en particulier les phases inspiratoires dudit patient pendant le traitement, est représentée par un animal, à savoir ici un caméléon 31. Bien entendu, tout autre contenant et/ou autre animal ou personnage peuvent convenir.

Plus précisément, afin d'assister une personne n'ayant pas ou peu de connaissances médicales, par exemple une auxiliaire de vie ou une personne de l'entourage familial du patient (les aidants), dans la mise en oeuvre d'un appareil à toux 10 selon l'invention de manière à opérer au moins des insufflations de gaz à un patient souffrant de troubles respiratoires, les moyens de pilotage 16 commandent l'affichage sur l'écran d'affichage 20, dans le mode de réalisation proposé en exemple, à savoir ici en mode IPPB, de la vidéo d'animation mettant en scène ici un animal, à savoir ici un caméléon 31, en tant que représentation graphique 30 symbolisant le patient et ses phases respiratoires (i.e. inspiratoire et expiratoire), et préférentiellement une phase de pause optionnelle.

Dans cette animation vidéo, une représentation d'un flux de gaz 32 symbolise le débit de gaz insufflé au caméléon 31. Bien entendu, des représentations graphiques additionnelles ou différentes sont possibles.

On voit aussi que l'écran 20 comprend un fond d'écran 33 comprenant un décor ou analogue, à savoir ici une représentation de paysage de montagnes. Le caméléon 31 et la ou les représentations graphiques additionnelles 32, viennent se superposer audit paysage afin de créer une ambiance agréable et ludique pour l'utilisateur.

L'appareil à toux 10 comprend par ailleurs des moyens de mémorisation 17, telle qu'une carte mémoire, par exemple une EEPROM ou directement dans un exécutable (i.e. programme de processeur) ou autre, pour mémoriser la vidéo d'animation (i.e. animation vidéo) et/ou d'autres paramètres, en particulier les préréglages pour les modes INEX ou IPPB.

Les moyens de pilotage 16 sont donc configurés pour aller retrouver dans les moyens de mémorisation 17 et ensuite afficher à l'écran 20, l'animation vidéo au début d'un traitement, en particulier au démarrage d'une phase d'insufflation de gaz, après sélection du mode de ventilation désiré, à savoir ici le mode IPPB, et ensuite activation (i.e. appui digital) par l'utilisateur d'une touche tactile 65 de démarrage/arrêt de traitement, comme détaillé ci-après.

Par ailleurs, les moyens de pilotage 16 sont aussi configurés pour aller retrouver au sein des moyens de mémorisation 17, les préréglages des modes ventilatoires INEX ou IPPB et ensuite les appliquer, c'est-à-dire commander par exemple la turbine 2, après sélection par l'utilisateur du mode désiré, typiquement après appui sur une touche de sélection de mode, par exemple IPPB, comme expliqué ci-après.

Après appui sur la touche tactile 65 de démarrage/arrêt de traitement, l'appareil se met en pause jusqu'au début d'une phase inspiratoire du patient qui peut être détectée par des moyens de détection d'inspiration comme expliqué ci-après, ou alors initiée par appui du patient ou d'un aidant sur la touche tactile 64 de démarrage de cycle servant à déclencher manuellement une phase d'insufflation par l'appareil 1.

En effet, l'appareil comprend des moyens de détection d'inspiration, tel un système déclencheur (« trigger » en anglais), configurés pour opérer un démarrage de l'insufflation de gaz, après détection d'une inspiration du patient, c'est-à-dire d'un début de phase inspiratoire, en utilisant par exemple un capteur de pression qui est agencé au niveau de la sortie patient et qui fournit des mesures de pression aux moyens de pilotage, typiquement un microprocesseur, qui les analysent pour en déduire le début d'une phase inspiratoire initiée par le patient.

Autrement dit, en mode IPPB :
- le démarrage de l'insufflation, selon le mode de réalisation, soit par déclenchement manuel, par exemple après appui sur la touche tactile 64, soit à l'aide de moyens de détection d'inspiration, tel un système déclencheur respiratoire, détectant un début d'inspiration de la part du patient lui-même, de préférence la sensibilité du système déclencheur est réglable.
- la phase d'inspiration se fait avec un débit réglable et se termine soit quand la pression maximale préfixée est atteinte, soit quand la durée maximale d'inspiration préréglée est atteinte.
- la phase d'expiration est passive afin que le patient puisse relâcher tout le gaz emmagasiné dans ses poumons. Eventuellement, un "soutien expiratoire" est mis en place, c'est à dire une légère résistance à l'expiration pour allonger le temps d'expiration du patient.
- la durée totale d'une séance de traitement peut être éventuellement réglée.

On voit par ailleurs sur Fig. 2, qui montre l'écran 20 avant le démarrage d'un traitement, que l'écran d'affichage 20 de l'IHM 19 comprend d'autres éléments, tels des moyens d'activation, de sélection ou analogue, en particulier des touches de sélection, notamment de préréglage permettent de sélectionner/activer, i.e. d'utiliser, des préréglages (appelées '*presets*' en anglais) préenregistrés, i.e. mémorisés, c'est-à-dire des réglages particuliers (pression, durées, pause, etc...) des modes ventilatoires INEX et IPPB, qui sont donc adaptés à certaines situations cliniques données, par exemple pour la nuit, pour le matin...

Par exemple, l'écran d'affichage 20 de l'IHM 19 comprend dans le mode de réalisation proposé :
- une touche tactile 60 de mise en route ou arrêt (OFF) de l'appareil.
- des touches tactiles 61 pour régler ou sélectionner des préréglages mémorisés adaptés aux modes de ventilation INEX et IPPB, par exemple des préréglages concernant la durée ou temps maximal d'insufflation et/ou la pression de consigne maximale en mode IPPB.
- une touche tactile 62 pour sélectionner un préréglage (preset) pour une ventilation en mode INEX AUTO, c'est-à-dire en mode INEX avec sous-mode de fonctionnement automatique (AUTO).
- une touche tactile 65 de démarrage/arrêt de traitement pour démarrer et/ou stopper le fonctionnement de la turbine 2.
- une touche tactile 63 pour accéder à différents menus ou sélections.
- une touche tactile 64 pour déclencher manuellement une inspiration, c'est-à-dire pour commencer une insufflation de gaz.
- un indicateur visuel coloré 62 de bon positionnement du masque. Si le masque est mal positionné et que l'appareil détecte une fuite trop importante, l'indicateur 62 s'allume à la fin de l'inspiration, par exemple en orange ou en une autre couleur.

L'écran d'affichage 20 permet aussi d'afficher, par exemple au sein d'une fenêtre 40 située en haut d'écran 20, donnant le volume d'air insufflé (valeur instantanée au cours de la phase d'inspiration), le volume cible de la séance (déterminé sur la base de la moyenne des 3 premières inspirations de la séance) et le nombre de cycles de respiration effectués depuis le début de la séance de traitement du patient.

Enfin, l'écran d'affichage 20 comprend aussi, dans le mode de réalisation proposé, un bandeau haut 41, dans lequel sont affichées différentes informations utiles comme le rappel du mode (ici le mode IPPB par exemple) ayant été sélectionné, une icône d'autonomie de la batterie électrique (ici 82%), une icône de transmission sans fil, par exemple en wifi, la date, l'heure.... ou autres.

Fig. 3 schématise l'affichage sur l'écran d'affichage 20 lors d'une insufflation de gaz montrant l'animation vidéo mis en oeuvre par les moyens de pilotage 16 comprenant le caméléon 31.

Comme on peut le voir, sur la Fig. 2, le caméléon 31 est représenté (i.e. forme/aspect) totalement dégonflé, donc avec des dimensions minimales, alors qu'à l'inverse, le caméléon 41 est représenté gonflé de gaz sur la Fig. 3.

Au début d'une phase d'insufflation de gaz, i.e. d'air, les moyens de pilotage 16 commandent l'affichage sur l'écran 20 de l'animation vidéo, après appui par l'utilisateur sur une touche tactile 64 de démarrage d'inhalation, de manière à montrer le caméléon 31 se gonflant ou grossissant progressivement, c'est-à-dire que ses dimensions et son aspect/sa forme augmentent à l'écran 20, mimant ainsi une entrée progressive de gaz (i.e. comme le font les poumons du patient).

Préférentiellement, lors d'une phase d'exsufflation de gaz, l'animation vidéo montre l'inverse, à savoir le caméléon 41, i.e. représentant le patient pendant son traitement, qui se dégonfle ou s'amincit, mimant ainsi une sortie de gaz des poumons du patient.

Le déroulé de l'affichage de la vidéo d'animation est synchronisé uniquement avec les volumes de gaz, lors des phases d'insufflation de gaz en mode IPPB. Il ne dépend d'aucune mesure de paramètre patient, en particulier d'aucun paramètre thoracique ou analogue, contrairement à l'art antérieur.

Plus précisément, l'appareil 10 comprend un capteur de débit permettant de déterminer le débit de gaz fourni par la turbine 1, lequel est agencé sur le trajet du gaz en aval de la sortie de la turbine 1, typiquement sur la ligne d'insufflation 4.

Les mesures opérées par le capteur de débit sont traitées par les moyens de pilotage 16 afin d'en déduire le volume de gaz délivré par la turbine 1.

Sur les Figures, le flux gazeux 32 va vers le caméléon 31 (cf. Fig. 3) pour schématiser la circulation du gaz en direction du patient ou dans le sens opposé pour schématiser la sortie de gaz des poumons du patient. Sur la Fig. 2, l'absence d'affichage de flux gazeux 32 montre que le traitement n'a pas encore débuté.

Pendant une éventuelle phase de pause (non montrée) suivant une phase d'exsufflation de gaz, l'animation vidéo montre un caméléon 31 non-évolutif, c'est-à-dire qu'avec forme, dimensions et aspect qui ne changent pas à l'écran 20 étant donné qu'aucun échange gazeux ne se produit entre eux.

Autrement dit, de façon schématique, l'écran 20 affiche une animation vidéo qui montre:
- pendant chaque inspiration/insufflation (cf. Fig. 3), un caméléon 31 (i.e. patient) qui gonfle et change de couleur, passant par exemple du violet au vert en fonction du % du volume de gaz insufflé au patient,
- pendant chaque expiration/l'exsufflation, un caméléon 31 (i.e. patient) qui dégonfle, et
- pendant chaque pause éventuelle, un caméléon 31 qui ne varie pas.

En affichant de telles représentations sur l'écran 20, l'appareil à toux 10 procure une aide visuelle très utile au patient et à la personne qui l'assiste lors de la mise en oeuvre de son traitement. Ainsi, le caméléon varie (dimensions, couleurs...) en fonction du volume de gaz fourni par la turbine, donc indirectement de l'effort fourni par le patient qui inspire le gaz. L'animation encourage donc le patient à gonfler le plus possible ses poumons en inhalant si possible tout le volume gazeux qui lui est apporté pendant chaque phase inspiratoire.

L'animation vidéo mise en oeuvre sur l'écran 20 est ici une véritable solution technique au problème susmentionné. En effet, en procédant ainsi, le patient est assuré de bien réaliser son traitement, c'est-à-dire d'obtenir un traitement efficace, en particulier pour améliorer son élasticité pulmonaire et/ou thoracique, et ce, sans nécessiter la présence sur place d'un personnel soignant qualifié, de type kinésithérapeute ou analogue.

Dans l'appareil à toux 10 selon l'invention, l'affichage de l'animation vidéo et son déroulé dépendent uniquement du volume de gaz envoyé au patient.

Lorsque le patient a bien réalisé son traitement, c'est-à-dire à bien calqué sa respiration sur les phases d'insufflations et d'exsufflation de l'appareil à toux, les moyens de pilotage 16 peuvent être en outre aussi configurés pour afficher sur l'écran d'affichage 20, une animation vidéo additionnelle symbolisant cette bonne réalisation de son traitement par le patient, par exemple une animation vidéo additionnelle du type pluie de confetti ou une récompense comme une coupe ou un trophée, ou autre.

L'appareil d'assistance à la toux ou appareil à toux selon l'invention permet d'opérer efficacement des insufflations de gaz à un patient souffrant de troubles respiratoires nécessitant une aide à l'évacuation des sécrétions pulmonaires et/ou pour améliorer son élasticité pulmonaire et/ou thoracique, même lorsque le patient est assisté par une personne n'ayant pas ou peu de connaissances médicales, par exemple une auxiliaire de vie ou une personne de l'entourage familial du patient, encore appelés un « aidant », puisqu'il lui suffit de calquer sa respiration sur l'animation vidéo qui s'affiche à l'écran.

## Revendications

1. Appareil à toux (10) pour opérer au moins une insufflation de gaz à un patient, comprenant :
- une turbine motorisée (1) en communication fluidique avec un circuit de gaz (4, 5, 6) comprenant une ligne d'insufflation (4) et une ligne d'exsufflation (5),
- un écran d'affichage (20),
- et des moyens de pilotage (16) configurés pour commander la turbine (1) et un affichage sur l'écran d'affichage (20), lesdits moyens de pilotage (16) étant configurés pour afficher sur l'écran d'affichage (20), une animation vidéo mettant en oeuvre une représentation graphique (30) symbolisant un patient pendant au moins une phase d'insufflation de gaz, où
- il comprend un capteur de débit agencé sur le trajet du gaz en aval de la sortie de la turbine (1) et configuré pour opérer des mesures permettant de déterminer le débit de gaz fourni par la turbine (1), lesdites mesures opérées par le capteur de débit étant traitées par les moyens de pilotage afin d'en déduire un volume de gaz délivré par la turbine (1),
- la forme, l'aspect et/ou les dimensions de ladite représentation graphique (30) affichée sur l'écran d'affichage (20) varient en corrélation avec le volume de gaz fourni par la turbine (1) au patient, pendant ladite au moins une phase d'insufflation de gaz, et
- les moyens de pilotage (16) sont configurés pour :
a) commander un démarrage de la turbine (2) et simultanément de l'animation vidéo en réponse à une activation par l'utilisateur de moyens de démarrage d'insufflation (64) ou à une détection d'une inspiration de l'utilisateur, et
b) stopper la turbine et simultanément l'animation vidéo lorsqu'une pression maximale d'insufflation est atteinte ou à la fin d'une durée maximale d'insufflation.

2. Appareil selon la revendication 1, **caractérisé en ce que** les variations de dimensions de la représentation graphique (30) se font de manière progressive entre le début et la fin de la phase d'insufflation.

3. Appareil selon l'une des revendications 1 ou 2, **caractérisé en ce que** la représentation graphique (30) représente ou comprend un animal ou un personnage, en particulier un caméléon (31).

4. Appareil selon la revendication 1, **caractérisé en ce qu'**il comprenant en outre des moyens de sélection d'un mode de ventilation choisi parmi les modes INEX et IPPB, où :
- le mode INEX correspond à une alternance de phases insufflatoire et exsufflatoire de gaz avec insufflation de gaz à pression positive suivi d'une exsufflation de gaz par mise en dépression, et
- le mode IPPB correspond à une alternance de phases insufflatoire et exsufflatoire de gaz avec insufflation de gaz jusqu'à atteindre une pression de consigne maximale ou une durée d'insufflation maximale, suivi d'une exsufflation de gaz sans mise en dépression.

5. Appareil selon la revendication 4, **caractérisé en ce qu'**il comprend des moyens de mémorisation configurés pour mémoriser des préréglages correspondant à des modes de ventilation INEX et IPPB.

6. Appareil selon la revendication 5, **caractérisé en ce que** les moyens de pilotage (16) sont en outre configurés pour retrouver au sein des moyens de mémorisation, au moins un préréglage donné adapté à la mise en oeuvre d'un mode de ventilation INEX ou IPPB en réponse à une sélection par un utilisateur, via les moyens de sélection de mode de ventilation, d'un desdits modes de ventilation INEX ou IPPB.

7. Appareil selon la revendication 1, **caractérisé en ce que** les variations de dimensions de la représentation graphique se font de manière synchronisées avec une variation de couleur de la représentation graphique.

8. Appareil selon les revendications 1 et 4, **caractérisé en ce que** l'écran d'affichage (20) est configuré pour afficher en outre, en mode IPPB, un volume de gaz courant, un volume de gaz cible, une durée de séance et/ou un nombre de cycle effectués.

9. Appareil selon les revendications 1 et 4, **caractérisé en ce que** pendant une ou chaque phase d'insufflation en mode IPPB, la turbine (1) est commandée pour générer une pression positive (P+) comprise entre 5 et 70 mbar (pression relative par rapport à la pression atmosphérique).

10. Appareil selon les revendications 1 ou 4, **caractérisé en ce que** les moyens de pilotage (16) sont configurés pour stopper la turbine et simultanément l'animation vidéo lorsqu'une pression maximale d'insufflation est atteinte ou à la fin d'une durée maximale d'insufflation, en mode IPPB.

11. Appareil selon la revendication 1, **caractérisé en ce que** la forme, l'aspect et/ou les dimensions de la représentation graphique (30) affichée sur l'écran d'affichage (20) varient uniquement en corrélation avec le volume de gaz fourni par la turbine (1) au patient.

12. Appareil selon l'une des revendications 1 ou 11, **caractérisé en ce que** les dimensions de la représentation graphique (30) affichée sur l'écran d'affichage (20) varient proportionnellement au volume de gaz fourni par la turbine (1) au patient.

13. Appareil selon la revendication 1, **caractérisé en ce que** le capteur de débit est agencé sur la ligne d'insufflation (4).

## Patentansprüche

1. Hustengerät (10) zum Vornehmen mindestens einer Insufflation von Gas bei einem Patienten, umfassend:
- eine motorbetriebene Turbine (1) in Fluidverbindung mit einem Gaskreislauf (4, 5, 6), der eine Insufflationsleitung (4) und eine Exsufflationsleitung (5) umfasst,
- einen Anzeigebildschirm (20),
- und Ansteuermittel (16), die dafür ausgelegt sind, die Turbine (1) und eine Anzeige auf dem Anzeigebildschirm (20) zu steuern, wobei die Ansteuermittel (16) dafür ausgelegt sind, auf dem Anzeigebildschirm (20) eine Videoanimation anzuzeigen, bei der eine grafische Darstellung (30) verwendet wird, die einen Patienten während mindestens einer Gasinsufflationsphase symbolisiert,
wobei
- es einen Durchflusssensor umfasst, der im Strömungsweg des Gases stromabwärts des Auslasses der Turbine (1) angeordnet ist und dafür ausgelegt ist, Messungen durchzuführen, welche ermöglichen, die von der Turbine (1) gelieferte Gasdurchflussmenge zu bestimmen, wobei die von dem Durchflusssensor durchgeführten Messungen von den Ansteuermitteln verarbeitet werden, um daraus ein von der Turbine (1) geliefertes Gasvolumen abzuleiten,
- die Form, das Aussehen und/oder die Abmessungen der grafischen Darstellung (30), die auf dem Anzeigebildschirm (20) angezeigt wird, im Zusammenhang mit dem Gasvolumen variieren, das von der Turbine (1) während der mindestens einen Gasinsufflationsphase dem Patienten zugeführt wird, und
- die Ansteuermittel (16) dafür ausgelegt sind:
a) ein Starten der Turbine (2) und gleichzeitig der Videoanimation in Reaktion auf eine Aktivierung von Mitteln zum Starten einer Insufflation (64) durch den Benutzer oder eine Detektion einer Einatmung des Benutzers anzusteuern, und
b) die Turbine und gleichzeitig die Videoanimation zu stoppen, wenn ein maximaler Insufflationsdruck erreicht ist, oder am Ende einer maximalen Insufflationsdauer.

2. Gerät nach Anspruch 1, **dadurch gekennzeichnet, dass** die Änderungen von Abmessungen der grafischen Darstellung (30) zwischen dem Beginn und dem Ende der Insufflationsphase schrittweise erfolgen.

3. Gerät nach einem der Ansprüche 1 oder 2, **dadurch gekennzeichnet, dass** die grafische Darstellung (30) ein Tier oder eine Figur darstellt, insbesondere ein Chamäleon (31).

4. Gerät nach Anspruch 1, **dadurch gekennzeichnet, dass** es außerdem Mittel zur Auswahl eines Beatmungsmodus aus dem Modi INEX und IPPB umfasst, wobei:
- der Modus INEX einem Wechsel von Gasinsufflations- und -exsufflationsphasen entspricht, mit Insufflation von Gas mit Überdruck, gefolgt von einer Exsufflation von Gas durch Erzeugung von Unterdruck, und
- der Modus IPPB einem Wechsel von Gasinsufflations- und -exsufflationsphasen entspricht, mit Insufflation von Gas, bis ein maximaler Solldruck oder eine maximale Insufflationsdauer erreicht ist, gefolgt von Exsufflation von Gas ohne Erzeugung von Unterdruck.

5. Gerät nach Anspruch 4, **dadurch gekennzeichnet, dass** es Speichermittel umfasst, die dafür ausgelegt sind, Voreinstellungen zu speichern, die Beatmungsmodi INEX und IPPB entsprechen.

6. Gerät nach Anspruch 5, **dadurch gekennzeichnet, dass** die Ansteuermittel (16) außerdem dafür ausgelegt sind, in Reaktion auf eine Auswahl eines der Beatmungsmodi INEX oder IPPB über die Mittel zur Auswahl eines Beatmungsmodus durch einen Benutzer in den Speichermitteln mindestens eine gegebene Voreinstellung abzurufen, die zur Durchführung eines Beatmungsmodus INEX oder IPPB geeignet ist.

7. Gerät nach Anspruch 1, **dadurch gekennzeichnet, dass** die Änderungen von Abmessungen der grafischen Darstellung synchronisiert mit einer Farbänderung der grafischen Darstellung erfolgen.

8. Gerät nach einem der Ansprüche 1 bis 4, **dadurch gekennzeichnet, dass** der Anzeigebildschirm (20) dafür ausgelegt ist, außerdem im Modus IPPB ein aktuelles Gasvolumen, ein Zielgasvolumen, eine Behandlungsdauer und/oder eine Anzahl durchgeführter Zyklen anzuzeigen.

9. Gerät nach einem der Ansprüche 1 bis 4, **dadurch gekennzeichnet, dass** während einer oder jeder Insufflationsphase im Modus IPPB die Turbine (1) so angesteuert wird, dass sie einen Überdruck (P+) zwischen 5 und 70 mbar (relativer Druck in Bezug auf den atmosphärischen Druck) erzeugt.

10. Gerät nach einem der Ansprüche 1 bis 4, **dadurch gekennzeichnet, dass** die Ansteuermittel (16) dafür ausgelegt sind, im Modus IPPB die Turbine und gleichzeitig die Videoanimation zu stoppen, wenn ein maximaler Insufflationsdruck erreicht ist, oder am Ende einer maximalen Insufflationsdauer.

11. Gerät nach Anspruch 1, **dadurch gekennzeichnet, dass** die Form, das Aussehen und/oder die Abmessungen der grafischen Darstellung (30), die auf dem Anzeigebildschirm (20) angezeigt wird, sich ausschließlich im Zusammenhang mit dem Gasvolumen ändern, das von der Turbine (1) dem Patienten zugeführt wird.

12. Gerät nach Anspruch 1 oder 11, **dadurch gekennzeichnet, dass** die Abmessungen der grafischen Darstellung (30), die auf dem Anzeigebildschirm (20) angezeigt wird, sich proportional zu dem Gasvolumen ändern, das von der Turbine (1) dem Patienten zugeführt wird.

13. Gerät nach Anspruch 1, **dadurch gekennzeichnet, dass** der Durchflusssensor an der Insufflationsleitung (4) angeordnet ist.

## Claims

1. Cough assist device (10) for performing at least one insufflation of gas to a patient, comprising:
- a motorized turbine (1) in fluidic communication with a gas circuit (4, 5, 6) comprising an insufflation line (4) and an exsufflation line (5),
- a display screen (20),
- and control means (16) configured to control the turbine (1) and a display on the display screen (20), said control means (16) being configured to display, on the display screen (20), a video animation implementing a graphical representation (30) symbolizing a patient during at least one phase of insufflation of gas,
where
- it comprises a flow rate sensor arranged on the path of the gas downstream of the outlet of the turbine (1) and configured to carry out measurements making it possible to determine the gas flow rate supplied by the turbine (1), said measurements carried out by the flow rate sensor being processed by the control means in order to deduce therefrom a volume of gas delivered by the turbine (1),
- the shape, the appearance and/or the dimensions of said graphical representation (30) displayed on the display screen (20) vary in correlation with the volume of gas supplied to the patient by the turbine (1), during said at least one phase of insufflation of gas, and
- the control means (16) are configured to:
a) trigger a start-up of the turbine (2), and simultaneously of the video animation, in response to an activation, by the user, of insufflation start-up means (64) or to a detection of an inspiration by the user, and
b) stop the turbine, and simultaneously the video animation, when a maximum insufflation pressure is reached or at the end of a maximum duration of insufflation.

2. Device according to Claim 1, **characterized in that** the variations in dimensions of the graphical representation (30) take place progressively between the start and the end of the insufflation phase.

3. Device according to either of Claims 1 and 2, **characterized in that** the graphical representation (30) represents or comprises an animal or a character, in particular a chameleon (31).

4. Device according to Claim 1, **characterized in that** it also comprises means for selecting a ventilation mode chosen from between the INEX and IPPB modes, where:
- the INEX mode corresponds to an alternation of insufflatory and exsufflatory phases of gas, with insufflation of gas at positive pressure followed by an exsufflation of gas by application of negative pressure, and
- the IPPB mode corresponds to an alternation of insufflatory and exsufflatory phases of gas, with insufflation of gas until a maximum setpoint pressure or a maximum duration of insufflation is reached, followed by an exsufflation of gas without application of negative pressure.

5. Device according to Claim 4, **characterized in that** it comprises storage means configured to store presets corresponding to INEX and IPPB ventilation modes.

6. Device according to Claim 5, **characterized in that** the control means (16) are additionally configured to find, within the storage means, at least one given preset adapted to the implementation of an INEX or IPPB ventilation mode in response to a selection by a user, via the ventilation mode selection means, of one of said ventilation modes INEX or IPPB.

7. Device according to Claim 1, **characterized in that** the variations in dimensions of the graphical representation take place in a manner synchronized with a variation in colour of the graphical representation.

8. Device according to Claims 1 and 4, **characterized in that** the display screen (20) is configured to additionally display, in IPPB mode, a current gas volume, a target gas volume, a session duration and/or or a number of cycles performed.

9. Device according to Claims 1 and 4, **characterized in that**, during one or each insufflation phase in IPPB mode, the turbine (1) is controlled to generate a positive pressure (P+) of between 5 and 70 mbar (relative pressure with respect to atmospheric pressure).

10. Device according to Claims 1 and 4, **characterized in that** the control means (16) are configured to stop the turbine, and simultaneously the video animation, when a maximum insufflation pressure is reached or at the end of a maximum duration of insufflation, in IPPB mode.

11. Device according to Claim 1, **characterized in that** the shape, the appearance and/or the dimensions of the graphical representation (30) displayed on the display screen (20) vary only in correlation with the volume of gas supplied to the patient by the turbine (1).

12. Device according to either of Claims 1 and 11, **characterized in that** the dimensions of the graphical representation (30) displayed on the display screen (20) vary in proportion to the volume of gas supplied to the patient by the turbine (1).

13. Device according to Claim 1, **characterized in that** the flow rate sensor is arranged on the insufflation line (4) .
